# EUROPÄISCHE PATENTSCHRIFT

(11) **EP 2 506 929 B1**
(45) Veröffentlichungstag und Bekanntmachung des Hinweises auf die Patenterteilung: **25.04.2018**
(21) Anmeldenummer: 10798982.4
(22) Anmeldetag: 02.12.2010
(51) Int. Cl.: A61N 5/10

(54) **BESTRAHLUNGSVORRICHTUNG**
IRRADIATION DEVICE
DISPOSITIF D'IRRADIATION

(30) Priorität: 05.12.2009 DE 102009058294
(43) Veröffentlichungstag der Anmeldung: 10.10.2012
(73) Patentinhaber: GSI Helmholtzzentrum für Schwerionenforschung GmbH, 64291 Darmstadt (DE)
(72) Erfinder: LÜCHTENBORG, Robert, 64827 Darmstadt (DE); BERT, Christoph, 63741 Aschaffenburg (DE); RICHTER, Daniel, 64289 Darmstadt (DE)
(74) Vertreter: Blumbach · Zinngrebe Patent- und Rechtsanwälte
(86) Internationale Anmeldenummer: PCT/EP2010/068716
(87) Internationale Veröffentlichungsnummer: WO 2011/067324

(56) Entgegenhaltungen:
- WO-A1-2011/006731
- WO-A1-2011/006737
- DE-A1-102007 045 879
- DE-B3-102006 024 243
- US-A1- 2007 140 425
- US-A1- 2008 002 811
- US-A1- 2008 049 896
- US-A1- 2009 147 916
- US-A1- 2009 161 827
- ENGHARDT ET AL: "Dose quantification from in-beam positron emission tomography", RADIOTHERAPY AND ONCOLOGY, ELSEVIER, IRELAND, vol. 73, 1 December 2004 (2004-12-01), pages S96-S98, XP005050411, ISSN: 0167-8140, DOI: 10.1016/S0167-8140(04)80024-0

## Beschreibung

Die Erfindung betrifft eine Bestrahlungsvorrichtung zur Bestrahlung von Gegenständen mit einem Scanningverfahren mit einem Hadronenstrahl und umfassend eine Steuervorrichtung sowie eine Bewegungsmessvorrichtung, wobei der Gegenstand zumindest ein zu bestrahlendes Zielvolumen sowie zumindest ein zu schützendes Volumen aufweist.

Eine Bestrahlung von Gegenständen wird zwischenzeitlich in unterschiedlichsten Bereichen der Technik durchgeführt. Je nach konkretem Einsatzerfordernis werden dabei auch unterschiedlichste Bestrahlungsverfahren sowie unterschiedlichste Bestrahlungsarten verwendet.

So ist es in manchen Gebieten der Technik erforderlich, Gegenstände flächig und möglichst gleichmäßig zu bestrahlen.

In anderen Gebieten der Technik ist es dagegen erforderlich, bestimmte Teile des zu bestrahlenden Gegenstands mit einer bestimmten, in der Regel besonders hohen Dosis zu bestrahlen, während die übrigen Teile des Gegenstands nicht bzw. nur wenig bestrahlt werden sollten. Ein Beispiel hierfür ist beispielsweise die Strukturierung von Mikroprozessoren unter Verwendung von elektromagnetischer Strahlung (zum Teil bis in den Röntgenbereich hinein) sowie bildgebender Masken.

In wieder anderen Gebieten der Technik ist die Dosisverteilung der Bestrahlung nicht nur in einer zweidimensionalen Ebene zu strukturieren, sondern vielmehr in allen drei Raumdimensionen. Gegebenenfalls ist auch eine dreidimensional strukturierte Bestrahlung mit zeitlicher Variation vorzunehmen (sogenannte vierdimensionale strukturierte Bestrahlung). Mit solchen Bestrahlungsverfahren ist es möglich, in einem bestimmten Volumenbereich, der sich innerhalb eines zu bestrahlenden Gegenstands befindet, eine bestimmte, relativ hohe Dosis einzubringen. Der das zu bestrahlende Zielvolumen umgebende Bereich des Gegenstands kann dagegen mit einer vergleichsweise niedrigen Dosis beaufschlagt werden. Beispiele für eine derartige Behandlung von Gegenständen sind beispielsweise in den Materialwissenschaften, bei der Herstellung hochintegrierter Bauteile (insbesondere Mikroprozessoren und Speicherchips) sowie bei der Herstellung von nanostrukturierten Mechaniken zu finden.

Ein weiteres Gebiet der Technik, bei dem derartige dreidimensionale bzw. vierdimensionale Bestrahlungsverfahren eingesetzt werden, liegt in der Medizintechnik. Hier ist es ebenfalls erforderlich, bestimmte Volumenbereiche innerhalb eines Körpers, wie beispielsweise Tumore, mit einer möglichst hohen Dosis zu beaufschlagen, wohingegen das umgebende Gewebe möglichst wenig bzw. vorzugsweise überhaupt nicht mit einer Dosis belastet werden sollte. Dies gilt in besonderem Maße, wenn es sich bei dem umgebenden Gewebe um ein Gewebe, wie beispielsweise ein oder mehrere kritische Organe (fachsprachlich meist als OAR für "Organ at risk" bezeichnet) handelt. Bei einem derartigen kritischen Geweben kann es sich beispielsweise um das Rückenmark, um Hauptblutgefäße (beispielsweise Aorta) oder Nervenknoten handeln.

Eine derartige erwünschte selektive Bestrahlung "in der Tiefe" des zu bestrahlenden Gegenstands kann beispielsweise dadurch realisiert werden, dass die Bestrahlung aus unterschiedlichsten Richtungen erfolgt, wobei sich sämtliche, aus unterschiedlichen Richtungen kommende Strahlen, in einem bestimmten Punkt bzw. in einem bestimmten Zielgebiet des zu bestrahlenden Gegenstands schneiden. Hierdurch kommt es zu einer hohen Gesamtdosis im Schnittpunkt der unterschiedlich gerichteten Strahlen, während die Dosis außerhalb dieses Schnittpunkts vergleichsweise niedrig ist.

Eine weitere Vorgehensweise, um eine derartige selektive Bestrahlung "in der Tiefe" eines Gegenstands zu realisieren besteht darin, dass bestimmte Bestrahlungsarten gewählt werden, die beim Durchgang durch Material eine Energieverlustcharakteristik aufweisen, die einen möglichst ausgeprägten Peak aufweist. Hier sind insbesondere Proton, Ionen und Schwerionen zu nennen. Diese weisen beim Durchgang durch Materie initial einen relativ geringen Energieverlust pro Längeneinheit auf, sodass die dort deponierte Strahlendosis relativ gering ist. Der Hauptteil der Strahlenergie wird vielmehr im sogenannten Bragg-Peak deponiert, sodass die dort in den zu bestrahlenden Gegenstand eingebrachte Dosis sehr hoch ist. Dadurch kann ein relativ scharf umrissener Zielpunkt "in der Mitte" eines zu bestrahlenden Gegenstands erreicht werden. Die Abmessungen eines derartigen Zielpunkts (bzw. eines bestimmten Volumenelements, fachsprachlich als sogenanntes "Voxel" bezeichnet) können beispielsweise im Bereich von nur 1 mm³ liegen. Ein zu bestrahlendes Zielvolumen mit einer bestimmten Konturierung kann dann beispielsweise durch Scanningverfahren, vorzugsweise durch sogenannte Rasterscanningverfahren, selektiv bestrahlt werden. Hierbei kann das Zielvolumen in sogenannte Rasterpunkte aufgeteilt werden. Der Teilchenstrahl (unter Berücksichtigung des Bragg-Peaks) wird dabei sukzessive über das zu bestrahlende Zielvolumen geführt. Eine Ablenkung in der X-Y-Ebene (eine sogenannte Isoenergieebene) kann durch Scannermagneten, die den Teilchenstrahl lateral ablenken können, durchfahren werden. Eine Variation in der Tiefe wird durch eine Veränderung der Energie des Teilchenstrahls, und damit durch eine Repositionierung des Bragg-Peaks erzielt. Während bei "klassischen" Scan-Verfahren der Teilchenstrahl im Wesentlichen kontinuierlich über das Zielvolumen verfahren wird, wird der Teilchenstrahl beim Rasterscanningverfahren jeweils auf ein Rasterpunkt oder Voxel gelenkt, wo er eine gewisse Zeit verharrt. Sobald in dem betreffenden Voxel eine bestimmte Dosis eingebracht wurde, wird das nächste Voxel angesteuert.

Auch wenn bei Verwendung von Schwerionen die in den Gegenstand eingebrachte Dosis relativ gut auf einen bestimmten Volumenbereich beschränkt werden kann, ist es auch hier unvermeidlich, dass auch Materie, welche insbesondere entlang des Teilchenstrahls vor oder hinter dem Zielpunkt liegt, und an sich nicht bestrahlt werden soll, mit einer gewissen Dosis beaufschlagt wird. Dies gilt insbesondere für die Bereiche, die vor dem zu bestrahlenden Zielgebiet liegen.

Derzeit verfügbare Bestrahlungsverfahren beinhalten in der Bestrahlungsplanung in der Regel die Schonung von Risikoorganen. Der Einfluss von während der Bestrahlung auftretender Bewegung kann jedoch oft nur unzureichend vorhergesagt werden. Eine Evaluation der tatsächlichen Dosisdepsoition im Zielvolumen und/oder insbesondere in Risikoorganen ist bei Bewegungseinfluss daher wenn überhaupt nur im Nachhinein möglich. Eine Intervention kann nur im Bezug auf eventuell noch folgende Fraktionen erfolgen. Dies ist jedoch problematisch, insbesondere dann, wenn sich der Tumor in einem kritischen Gewebebereich (beispielsweise in einem Organ wie der Lunge oder dem Herz) befindet, oder sich kritisches Gewebe in unmittelbarer Nähe zum Tumor befindet. Denn während bei "normalem" Gewebe eine gewisse, an sich unnötige Gewebezerstörung durchaus in Kauf genommen werden kann, so ist bei derartigem kritischen Gewebe (OAR) eine Schädigung desselben tunlichst zu vermeiden. Dies hat in der Vergangenheit oftmals dazu geführt, dass derartige, sich in bzw. in der Nähe von kritischem Gewebe befindliche Tumoren nicht oder bestenfalls mit starken Nebenwirkungen behandelt werden konnten.

Besonders problematisch war die Bestrahlung von Zielvolumen in beziehungsweise in der Nähe von empfindlichen Materialbereichen, wie beispielsweise von Tumoren in bzw. in der Nähe von kritischem Gewebe, insbesondere dann, wenn sich der betreffende Gegenstand bewegt, insbesondere auch in sich bewegt. Hierbei kann die das zu bestrahlende Zielvolumen umgebende Materie nicht nur translatorisch, sondern insbesondere auch rotatorisch und/oder deformatorisch bewegt werden. Dies hat zur Folge, dass beispielsweise bei einer veränderten Strahllage bezogen auf das Zielvolumen oder das zu schützedne Volumen (in Folge eines Scanverfahrens, aber auch bedingt durch Strahleinfallsrichtungsveränderung, beispielsweise bei Verwendung einer Gantry) die das Zielvolumen umgebende Materie beispielsweise durch die "in sich"-Bewegung im Bereich des einfallenden Teilchenstrahls, bestrahlt werden kann, insbesondere anders bestrahlt werden kann als dies in der voangegangenen Bestrahlungsplanung berücksichtigt wurde. Dies kann entsprechende Schädigungen des betreffenden Gewebes (der betreffenden Materie) zur Folge haben, was insbesondere dann problematisch ist, wenn es sich beispielsweise um besonders zu schützendes Gewebe (z.B. OAR) handelt.

Aus den Dokumenten US 2009/0161827 A1, US 2009/0147916 A1, US 2008/0049896 A1 und US 2008/0002811 A1 sind Röntgenbestrahlungssysteme bekannt.

Aus der DE 10 2007 045 879 A1 ist eine Ionenstrahlvorrichtung zur Bestrahlung eines bewegten Zielvolumens bekannt, wobei der Bestrahlungsplan ein Ensemble aus Rasterpunkten ist.

Die nicht vorveröffentlichten Dokumente WO 2011/006737 A1 sowie WO 2011/006731 (Art. 54(3) EPC) betreffen Bestrahlungsanlagen für sich bewegende Zielvolumen.

Die Aufgabe der Erfindung besteht darin, eine gegenüber dem Stand der Technik verbesserte Bestrahlungsvorrichtung zur Bestrahlung von Gegenständen vorzuschlagen.

Die vorliegende Erfindung löst diese Aufgabe. Die Aufgabe wird durch eine Bestrahlungsvorrichtung gemäß den Merkmalen des Anspruchs 1 gelöst.

Es wird eine Bestrahlungsvorrichtung zur Bestrahlung von Gegenständen mit einem Scanningverfahren mit einem Hadronenstrahl und umfassend eine Steuervorrichtung sowie eine Bewegungsmessvorrichtung vorgeschlagen, wobei der Gegenstand zumindest ein zu bestrahlendes Zielvolumen sowie zumindest ein zu schützendes Volumen aufweist, wobei für das zu bestrahlende Zielvolumen ein Zielraster und für das zu schützende Volumen ein Registrierungsraster vorgesehen sind, wobei für das zu schützende Volumen zumindest ein Signaldosiswert definiert ist, wobei die Bewegung des zu bestrahlenden Zielvolumens und des zu schützenden Volumens gemessen werden, wobei während der Bestrahlung des Gegenstands in jedem Volumenelement des Registrierungsrasters die in das zu schützende Volumen eingebrachte Dosis ermittelt wird und von der Steuervorrichtung zumindest ein Signal ausgegeben wird, sobald in zumindest einem Punkt des Registrierungsrasters die in das zu schützende Volumen eingebrachte Dosis zumindest einen Signaldosiswert überschreitet.

Bei dem zu schützenden Volumen kann es sich grundsätzlich um beliebige Volumenbereiche des zu bestrahlenden Gegenstands handeln, welche außerhalb des zu bestrahlenden Zielvolumens liegen. Insbesondere um eine ausreichend hohe Dosis innerhalb des zu bestrahlenden Zielvolumens (insbesondere an dessen Rändern) sicherstellen zu können, ist es jedoch auch möglich, dass unter dem zu schützenden Volumen ein Volumen zu verstehen ist, welches sich außerhalb eines sogenannten Sicherheitssaums befindet, wobei der Sicherheitssaum eine Schicht mit einer definierten Dicke darstellt, die um das zu bestrahlende Zielvolumen herum gelegt ist. Weiterhin kann es sich bei dem zu schützenden Volumen auch um einen Volumenbereich des Gegenstands handeln, der eine besonders große Empfindlichkeit gegenüber Strahlung hat und/oder aus anderweitigen Gründen (beispielsweise weil in dem entsprechenden Volumenbereich besonders kritische Elemente vorhanden sind) möglichst vor Strahlung zu schützen ist. Selbstverständlich ist es auch möglich, dass mehrere (beispielsweise zwei, drei, vier, fünf oder mehr) zu bestrahlende Zielvolumen und/oder zu schützende Volumen vorhanden sind, welche gegebenenfalls voneinander separiert sind. Zusätzlich oder alternativ ist es auch möglich, dass im Falle mehrerer zu schützender Volumen zwei, ein gewisser Anteil oder alle der zu schützenden Volumen unterschiedliche Signaldosiswerte aufweisen. Auf diese Weise kann der Bestrahlungsvorgang besonders gut auch für komplexe Strukturen angewendet werden. Insbesondere kann damit ein besonders weitgehender Schutz von empfindlichen Volumenbereichen, beispielsweise ein Schutz eines OAR, bei gleichzeitiger bestmöglicher Bestrahlung des zu bestrahlenden Volumens gewährleistet werden.

Bei der Art der Bestrahlung handelt es sich um einen Teilchenstrahl, welcher vorzugsweise eine relativ kleine Ausdehnung in einer, zwei bzw. mehreren senkrecht zur Strahlrichtung verlaufenden Richtungen aufweist. Besonders bevorzugt kann es sich um einen nadelscharfen Strahl handeln. Obgleich es üblicherweise bevorzugt ist, dass es sich bei dem Teilchenstrahl um einen "sortenreinen" Teilchenstrahl, insbesondere um einen Teilchenstrahl mit einer Ionensorte, beispielsweise Kohlenstoffionen, handelt, ist es auch denkbar, dass der Teilchenstrahl aus einem Gemisch zweier oder mehrerer unterschiedlicher Arten von Teilchen zusammengesetzt ist. Als Teilchen werden Hadronen eingesetzt. Insbesondere kann es sich um Pionen, Protonen, Ionen und/oder Schwerionen handeln. Unter Schwerionen werden üblicherweise Ionen verstanden, welche eine Kernladungszahl von ≥ 3 und/oder eine Kernmassenzahl von ≥ 5 aufweisen. Bei sämtlichen Strahlen ist es möglich, dass diese zumindest zeitweise und/oder zumindest teilweise zumindest quasi-kontinuierlich freigesetzt werden (also zumindest über gewisse Zeitdauern hinweg eine im Wesentlichen gleichbleibende Strahlintensität aufweisen). Ebenso ist es jedoch möglich, dass die Bestrahlung zumindest zeitweise und/oder zumindest teilweise unter Verwendung zumindest eines intensitätsmodulierten Strahls erfolgt.

Bei dem zu bestrahlenden Gegenstand kann es sich grundsätzlich um einen beliebigen Gegenstand, wie insbesondere ein Werkstück handeln, welches beispielsweise zumindest teilweise aus einem Halbleitermaterial bestehen kann. Ebenso ist es auch möglich, dass es sich bei dem Werkstück um ein sogenanntes "Phantom" handelt, welches bei medizintechnischen Vorrichtungen zur Simulation der Wirkung der technischen Vorrichtung auf einen Patienten eingesetzt wird. Derartige Phantome werden nicht nur für die Entwicklung der medizinischen Geräte, insbesondere Bestrahlungsvorrichtung verwendet, sondern insbesondere auch um deren Kalibrierung zu überprüfen bzw. zu erneuern und/oder um die korrekte Funktion des Geräts sicher zu stellen. Beispielsweise können derartige Phantome täglich am Beginn eines Behandlungstags eingesetzt werden, um Verletzungen oder gar Todesfälle durch funktionsuntüchtige Geräte nach Möglichkeit ausschließen zu können. Phantome können weiterhin zur Validierung eines Bestrahlungsplanes eingesetzt werden. Selbstverständlich kann es sich bei dem Gegenstand auch um ein Tier, eine Person oder allgemein um ein biologisches Gewebe handeln. Insbesondere bei einer Person und/oder bei einem Tier kann es sich bei dem zu schützenden Volumen um normales Gewebe und/oder ein kritisches Gewebe (z. B. Rückenmark) oder um ein Organ (insbesondere ein sogenanntes OAR (Organ at risk), wie beispielsweise die Lunge oder das Herz) handeln. Bei dem zu bestrahlenden Zielvolumen kann es sich insbesondere um einen Tumor handeln. Mit der vorgeschlagenen Bestrahlungsvorrichtung ist es insbesondere möglich, die in das/die zu schützende(n) Volumen eingebrachten Dosen kontinuierlich, also insbesondere auch während der Behandlung, insbesondere der Bestrahlung, bevorzugt im Wesentlichen während der gesamten Behandlung, kontrollieren, insbesondere überwachen und wenn notwendig, verändern zu können. Besonders bevorzugt ist die Überwachung der in das/die zu schützende(n) Volumen eingebrachten Dosis zumindest zeitweise und/oder zumindest bereichsweise auch ortsaufgelöst möglich. Dadurch kann die Bestrahlungsvorrichtung eine besonders große Sicherheit gewährleisten. Insbesondere kann zu jedem Zeitpunkt eine entsprechende, geeignete Maßnahme ergriffen werden, beispielsweise eine Änderung von Bestrahlungsparametern, wie beispielsweise Intensität des Teilchenstrahls, zumindest bei der Dosisdeposition in dedizierten Bereichen des zu bestrahlenden Volumens und/oder des zu schützenden Volumens, ergriffen werden, sobald ein Signaldosiswert erreicht wurde, bzw. droht, erreicht zu werden. Hierdurch kann ein weitestgehender Schutz eines Patienten, insbesondere in Bezug auf dessen kritische Gewebe realisiert werden. Im Stand der Technik wurde eine Überprüfung der in das zu schützende Volumen eingebrachten Dosis nämlich - falls überhaupt - nach Ablauf eines Behandlungsschritts (also beispielsweise im Falle eines Scanverfahrens erst nach dem vollständigen Durchscannen des zu schützenden Volumens) durchgeführt. Insbesondere beim Zusammentreffen mehrerer ungünstiger Randbedingungen kann dieser Zeitpunkt jedoch deutlich zu spät liegen, sodass beispielsweise ein kritischer Signaldosiswert bereits zum Teil massiv überschritten sein kann. Gegenüber derartigen, bekannten Bestrahlungsvorrichtungen weist daher die vorgeschlagene Bestrahlungsvorrichtung in aller Regel eine bedeutend höhere Sicherheit auf.

Insbesondere kann mit der vorliegenden Bestrahlungsvorrichtung online während der Bestrahlung auf beispielsweise durch Bewegung verursachte Änderungen der Dosisdeposition reagiert werden. Einer Überschreitung von Dosisgrenzwerten in zu schützenden Strukturen, insbesondere dem zu schützenden Volumen kann so vorgebeugt werden.

Bei dem zumindest einen Signaldosiswert kann es sich insbesondere um einen Warndosiswert und/oder um eine nicht zu überschreitende Maximaldosis handeln. Bei einem Warndosiswert kann es sich insbesondere um einen Wert handeln, bei dem nicht notwendigerweise sofort einschneidende Maßnahmen ergriffen werden müssen. Stattdessen kann bei einem derartigen Warndosiswert üblicherweise noch anderweitig reagiert werden, da noch eine gewisse zusätzliche Dosis eingebracht werden kann, ohne dass ein kritischer Fall eintritt. Beispielsweise kann beim Erreichen eines Warndosiswerts die noch folgende Bestrahlung geeignet geändert werden. Bei einer nicht zu überschreitenden Maximaldosis kann es sich dagegen um einen Wert handeln, welcher keinesfalls überschritten werden darf, da ansonsten einschneidende Konsequenzen, wie beispielsweise schwere Verletzungen die Folge sein können. Selbstverständlich ist es auch möglich, eine Mehrzahl von Signaldosiswerten, insbesondere auch eine Mehrzahl von Warndosiswerten vorzusehen (wobei dies insbesondere auch für einen und denselben Volumenbereich gelten kann), sodass basierend auf dem damit einhergehenden Gefährdungspotenzial unterschiedliche, jeweils angepasste Maßnahmen ergriffen werden können. Selbstverständlich können insbesondere beim Vorhandensein einer Mehrzahl von unterschiedlichen kritischen Volumina auch unterschiedliche Signalwerte bzw. unterschiedliche Sätze von Signalwerten definiert werden. Es ist darauf hinzuweisen, dass es sich bei den entsprechenden Signaldosiswerten nicht notwendigerweise um eine fixe Zahl (wie beispielsweise 5 Gy oder dergleichen) handeln muss. Vielmehr können die Signaldosiswerte auch beispielsweise einem sogenannten Dosis-Volumen-Histogramm (DVH) entnommen werden. Derartigen DVHs liegt die Erkenntnis zugrunde, dass für den Fall, dass ein zu schützendes Volumen flächenmäßig bestrahlt wird, eine geringere Grenzdosis zulässig ist, als in einem Fall, in dem ein zu schützendes Volumen nur in einem relativ kleinen Teilbereich (insbesondere punktuell) mit Strahlung beaufschlagt wird.

Insbesondere kann die Bestrahlungsvorrichtung derart ausgebildet sein, dass das ausgegebene Signal einen Abbruch des Bestrahlungsvorgangs bewirkt und/oder eine Unterbrechung des Bestrahlungsvorgangs bewirkt und/oder eine Änderung von zumindest Teilen des noch durchzuführenden Bestrahlungsvorgangs, wie insbesondere eine Verringerung der in zumindest Teilen des zu bestrahlenden Zielvolumens und/oder in zumindest Teilen des zu schützenden Volumens einzubringenden Dosis, bewirkt. Unter einer Unterbrechung des Bestrahlungsvorgangs ist insbesondere eine gewisse zeitliche Pause zu verstehen, welche in einem weiten Bereich, wie insbesondere in einem Bereich von Sekunden, Minuten, Stunden, Tagen, Wochen oder Monaten liegen kann. Diese Zeit kann beispielsweise bei der Therapie von Tumoren für eine zwischenzeitliche Erholung des Patienten verwendet werden, oder aber auch dazu, um die Vorrichtung, mit der die Bestrahlung durchgeführt wird, neu anzupassen. Beispielsweise kann die Zwischenzeit dazu genutzt werden, um eine Bestrahlungsplanung neu zu erstellen bzw. geeignet anzupassen, den Patienten neu auszurichten und/oder eine Gantry zu verstellen. Ebenso ist es auch möglich, dass die Bestrahlung kontinuierlich durchgeführt wird, und während der laufenden Bestrahlung beispielsweise online die Bestrahlungsplanung neu berechnet und/oder angepasst wird, oder dass während einer laufenden Bestrahlung der Patient neu im Verhältnis zum Teilchenstrahl justiert wird (beispielsweise durch eine Umlagerung des Patienten und/oder durch eine Bewegung einer Gantry). Ein Abbruch des Bestrahlungsvorgangs kann insbesondere dann sinnvoll sein, wenn der Wert einer nicht zu überschreitenden Maximaldosis erreicht, oder gar überschritten wurde. "Sanftere" Gegenmaßnahmen (wie zum Beispiel die Änderung von zumindest Teilen des noch durchzuführenden Bestrahlungsvorgangs und/oder die Unterbrechung und spätere Wiederaufnahme des Bestrahlungsvorgangs) sind insbesondere dann sinnvoll, wenn lediglich ein Warndosiswert erreicht oder überschritten wurde. Selbstverständlich können neben einer Änderung der Bestrahlungsplanung oder der Einfallsrichtung des Strahls auch andere Maßnahmen, wie beispielsweise ein sogenanntes "Gating", beispielsweise ein periodisches An- und Ausschalten des Teilchenstrahls, und/oder ein sogenanntes "Rescanning" mit geänderten Bestrahlungsparametern, ausgelöst werden.

Vorteilhaft ist es, wenn es sich bei der eingebrachten Dosis zumindest zeitweise und/oder zumindest teilweise um die applizierte Dosis und/oder um die deponierte Dosis handelt. Die applizierte Dosis wird üblicherweise dadurch bestimmt, dass die tatsächliche durchgeführte Bestrahlung gemessen wird (beispielsweise im Falle eines Teilchenstrahls durch Vermessung des Strahls, wie insbesondere durch Vermessung der Strahlposition und/oder der Strahlintensität und/oder der Strahlenergie) und die applizierte Dosis unter zumindest teilweiser Verwendung dieser Messwerte berechnet wird. Es fließen bei der Bestimmung der applizierten Dosis folglich in aller Regel Messwerte ein, wobei jedoch die eingebrachte Dosis üblicherweise nicht unmittelbar im zu bestrahlenden Gegenstand bestimmt wird, sondern lediglich mittelbar. Bei der deponierten Dosis wird dagegen üblicherweise die eingebrachte Dosis im zu bestrahlenden Gegenstand, insbesondere dem Zielvolumen und/oder dem zu schützendes Volumen, unmittelbar bestimmt, wozu beispielsweise implantierte Detektoren verwendet werden können, oder auch externe Detektoren (also nicht implantierte Detektoren), wie beispielsweise ein sogenannter PET (PET für Photonen-Emissions-Tomograph). Selbstverständlich ist es aber auch möglich, dass zumindest zeitweise und/oder zumindest teilweise die eingebrachte Dosis unter Verwendung der Steuerdaten der Bestrahlungsvorrichtung oder dergleichen ermittelt wird, unterstützt durch eine Bewegungsmessung des im Falle von bewegten Gegenständen, insbesondere eines bewegten Zielvolumens und/oder zu schützenden Volumens..

Besonders vorteilhaft kann die Bestrahlungsvorrichtung die Bestrahlung dann durchführen, wenn die Ermittlung der in das zu schützende Volumen eingebrachten Dosis und/oder die Ermittlung der in das zu bestrahlende Zielvolumen eingebrachten Dosis zumindest teilweise und/oder zumindest zeitweise unter Verwendung zumindest eines im betreffenden Volumen und/oder zumindest eines am Strahl gemessenen Messwerts erfolgt, wobei es sich bei dem am Strahl gemessenen Messwert insbesondere um die Strahlposition, die Strahlgröße, die Strahlform, die Strahlintensität und/oder die Strahlenergie handelt. Mit einer derartigen (zumindest teilweisen) Verwendung von aktuellen Messwerten kann die Genauigkeit der Bestrahlungsvorrichtung bei der Bestrahlung nochmals erhöht werden. Bei der Strahlintensität kann es sich insbesondere um die sogenannte integrierte Strahlintensität handeln. Bei der Ermittlung eines Messwerts im betreffenden Volumen kann in beliebiger Weise eine implantierte Messvorrichtung und/oder eine nicht-implantierte Messvorrichtung (wie beispielsweise ein Photonen-Emissions-Tomograph - PET) verwendet werden.

Die Bestrahlungsvorrichtung wird genutzt, wenn sich der zu bestrahlende Gegenstand zumindest zeitweise und/oder zumindest bereichsweise bewegt, insbesondere sich zumindest zeitweise und/oder zumindest bereichsweise in sich bewegt, wobei sich insbesondere das zu bestrahlende Zielvolumen und/oder das zu schützende Volumen zumindest zeitweise und/oder zumindest bereichsweise bewegen, speziell sich zumindest zeitweise und/oder zumindest bereichsweise relativ zueinander bewegen. Unter einer Bewegung ist dabei jeweils nicht nur eine translatorische Bewegung zu verstehen, sondern insbesondere auch eine rotatorische Bewegung und/oder eine Dehnungsbewegung des oder der betreffenden Volumenbereiche und/oder der sich gegebenenfalls dazwischen befindlichen Volumenbereiche. Derartige sich bewegende Volumina stellen besonders hohe Anforderungen an das Bestrahlungsverfahren bzw. die Bestrahlungsvorrichtung, wobei Dank des vorgeschlagenen Verfahrens ein besonders hoher Schutz des zu bestrahlenden Gegenstands (beispielsweise des Patienten, gegebenenfalls einschließlich dessen besonders zu schützender Gewebebereiche, insbesondere einschließlich dessen OARs) erzielt werden kann. Im Falle einer Bestrahlung eines Tumors eines Patienten können derartige Fälle beispielsweise dann auftreten, wenn sich der Tumor im Bereich der Lunge, im Bereich des Herzens und/oder im Bereich des Darms (oder jeweils in deren/dessen Nähe) befindet. Wenn rein beispielhaft ein Tumor (oder ein anderes so genanntes CTV für clinical target volume), der sich in der Lunge befindet, betrachtet wird, so bewegt sich dieser nicht nur, sondern er befindet sich darüber hinaus in der Nähe des Rückenmarks, sowie in der Nähe des sich bewegenden Mediastinums und gegebenenfalls auch in der Nähe des sich bewegenden Herzens, wobei es sich sowohl beim Rückenmark, beim Mediastinum und beim Herz um besonders kritische und daher besonders zu schonende Gewebe bzw. Organe handelt. Die Bestrahlungsvorrichtung ist derart ausgeführt, dass zumindest zeitweise und/oder zumindest bereichsweise die Position von zumindest Teilen des zu bestrahlenden Gegenstands gemessen wird, insbesondere unter zumindest teilweiser und/oder zumindest zeitweiser Verwendung bildgebender Verfahren. Bei den zu vermessenden Teilen des Gegenstands handelt es sich um das zumindest eine zu bestrahlende Zielvolumen und das zumindest eine zu schützende Volumen. Bei Kenntnis der Position von zumindest Teilen des zu bestrahlenden Gegenstands kann einerseits die Bestrahlung genauer einjustiert werden (insbesondere am Anfang des Bestrahlungsvorgangs) und darüber hinaus kann die während der Bestrahlung auftretende Bewegung des zu bestrahlenden Zielvolumens und/oder des zu schützenden Volumens registriert und gegebenenfalls berücksichtigt werden. Eine im Wesentlichen kontinuierliche Überwachung der Position (und damit der Bewegung) von zumindest Teilen des zu bestrahlenden Gegenstands (fachsprachlich "motion tracking" oder "tumor tracking" genannt) ist insbesondere für sogenannte "Motion Mitigation"-Verfahren, hier insbesondere für das fachsprachlich als "beam tracking"-Verfahren bezeichnete Verfahren vorteilhaft. Gegebenenfalls kann es sich bei dem Tracking-Verfahren auch um ein anderes Verfahren, wie beispielsweise das sogenannte "gating"-Verfahren handeln. Auch Kombinationen aus den genannten und gegebenenfalls weiteren Verfahren, wie beispielsweise Gating oder Rescanning sind denkbar.

Bei den durchgeführten Messungen der Bewegung ist es sinnvoll, wenn die Messwerte und/oder die aus den Messwerten gewonnenen Ergebnisse bei der aktuellen Bestrahlung und/oder bei nachfolgenden (Teil-)Bestrahlungsvorgängen berücksichtigt werden. Dies kann beispielsweise durch eine geänderte Strahlführung, ein geändertes Scanning, eine Anpassung der Bestrahlungsplanung oder dergleichen erfolgen. Hierdurch kann die Bestrahlungsqualität nochmals gegebenenfalls sogar deutlich verbessert werden.

Die Bestrahlungsvorrichtung ist derart ausgeführt, dass die Bestrahlung in Form eines Scanvorgangs, insbesondere eines Rasterscanvorgangs erfolgt. Ein Scanvorgang erfolgt dabei üblicherweise derart, dass sich der Ort, in den die Hauptdosis eingebracht wird, zumindest zeitweise im Wesentlichen kontinuierlich verändert. Gewisse Unterbrechungen sind dabei möglich, beispielsweise dann, wenn von einer "Bestrahlungszeile" in die nächste gewechselt wird und/oder die Isoenergieebene gewechselt wird. Bei einem Rasterscanvorgang wird dagegen der Strahl üblicherweise "ruckartig" von Bestrahlungspunkt zu Bestrahlungspunkt bewegt. Im jeweiligen Bestrahlungspunkt verharrt der Strahl üblicherweise eine gewisse Zeit, insbesondere solange, bis eine bestimmte (Teil-) Bestrahlungsdosis im betreffenden Punkt erreicht ist. Speziell ist es auch möglich, dass der Scanvorgang und/oder der Rasterscanvorgang zumindest zeitweise und/oder zumindest bereichsweise intensitätsmoduliert erfolgt. Lediglich der Vollständigkeit halber sollte darauf hingewiesen werden, dass es auch möglich ist, Scanvorgang und/oder Rasterscanvorgang nur zeitweise und/oder nur bereichsweise durchzuführen.

Eine weitere vorteilhafte Weiterbildung der Bestrahlungsvorrichtung kann sich ergeben, wenn die Bestrahlung in Form mehrerer Teilbestrahlungsvorgänge erfolgt, insbesondere in Form mehrerer Fraktionen und/oder mehrerer Rescanning-Vorgänge und/oder unter Verwendung von Gating-Verfahren. Unter einem Gating-Verfahren wird üblicherweise verstanden, dass der Teilchenstrahl moduliert ein- und ausgeschaltet wird in Abhängigkeit einer gemessenen oder vor der Bestrahlung bekannten Bewegung des Gegenstandes und/oder des Zielvolumens und/oder des zu schützenden Volumens. Ein Ein- bzw. Ausschalten kann beispielsweise dann erfolgen, wenn die Bewegung eines Tumors (oder eines sonstigen zu bestrahlenden Volumenbereichs) sich kurzfristig außerhalb des durch Strahlanpassung erreichbaren Bereichs bewegt. Insbesondere wird mit Gating aber eine Technik bezeichnet, die dadurch, dass der Behandlungsstrahl nur in bestimmten Bewegungszuständen des Tumors (z.B. Ende der Ausatmung) angeschaltet wird, den Einfluss der Bewegung auf die Bestrahlung vermindert.

Auftretende Ortsfluktuationen, die zu einem vorübergehenden Abschalten des Strahls führen können, können relativ selten sein, insbesondere dann, wenn Gating-Verfahren mit Tracking-Verfahren kombiniert werden. Bei sogenannten Rescanningvorgängen wird üblicherweise die gesamte, einzubringende Dosis in mehrere Teilvorgänge aufgeteilt, sodass sich Bewegungen des Tumors (des zu bestrahlenden Volumens) im statistischen Mittel ausmitteln. Unter Fraktionen ist üblicherweise die Aufteilung einer Behandlung (Bestrahlung) in mehrere zeitlich voneinander beabstandete Teilbestrahlungsvorgänge zu verstehen. Der zeitliche Abstand zwischen zwei Teilbestrahlungsvorgängen liegt beim fraktionierten Bestrahlen üblicherweise im Bereich von Stunden, bis Tagen, kann aber auch Wochen und/oder Monate betragen. Durch eine derartige Aufteilung in mehrere Teilbestrahlungsvorgänge kann das Verfahren insbesondere bei medizinischen Anwendungsgebieten oftmals effektiver und gegebenenfalls auch für den Patienten schonender durchgeführt werden.

Bei der Steuervorrichtung für die Bestrahlungsvorrichtung kann es sich in beliebiger Weise um eine Vorrichtung handeln, deren Funktionalitäten hardwaremäßig realisiert sind und/oder um eine Vorrichtung handeln, deren Funktionalitäten softwaremäßig realisiert sind. Auch Mischformen sind dabei denkbar, derart, dass manche Funktionalitäten hardwaremäßig realisiert sind, während manche Funktionalitäten softwaremäßig realisiert sind. Im Falle einer softwaremäßigen Realisierung kann die Steuervorrichtung einen oder mehrere Computer bzw. Computerkomponenten umfassen. Dabei kann es sich insbesondere um Personalcomputer (PCs), Großrechner, Workstations oder auch Einplatinencomputer handeln. Möglich ist es auch, dass die Rechenlast auf eine Mehrzahl von unterschiedlichen Computern bzw. Computerkomponenten aufgeteilt wird.

Insbesondere ist es möglich, dass die Bestrahlungsvorrichtung zumindest eine Messvorrichtung, insbesondere zumindest eine Strahlmessvorrichtung aufweist, wobei die Strahlmessvorrichtung insbesondere als Strahlpositionsmessvorrichtung, Strahlintensitätsmessvorrichtung und/oder Strahlenenergiemessvorrichtung ausgebildet sein kann. Insbesondere die Strahlmessvorrichtungen können dabei zumindest bereichsweise als Ionisationskammervorrichtungen und/oder als Vieldrahtkammern ausgebildet sein. Derartige Komponenten erhöhen üblicherweise die Bestrahlungsgenauigkeit der Bestrahlungsvorrichtung, wenn diese zur Bestrahlung eines Gegenstands verwendet wird. Gerade die vorgeschlagenen Komponenten haben dabei üblicherweise einen besonders großen Einfluss auf eine Verbesserung der Genauigkeit der Bestrahlung. Ionisationskammervorrichtungen sowie Vieldrahtkammern haben sich darüber hinaus für die ihnen übertragenen Messaufgaben bewährt und sind gut (gegebenenfalls auch kommerziell) erhältlich.

Bei der Beschleunigervorrichtung kann es sich insbesondere um einen Teilchenbeschleuniger handeln, der beispielsweise zumindest einen Linearbeschleuniger, zumindest ein Synchotron und/oder zumindest ein Zyklotron aufweisen kann. Auch Kombinationen der genannten (und weiterer) Beschleunigervorrichtungen sind denkbar. So weisen Synchotrone üblicherweise einen Linearbeschleuniger als Vorbeschleuniger auf. Die Beschleunigervorrichtung kann einen Teilchenstrahl mit den für die Bestrahlung des Zielvorlumens notwendigen Strahlparametern bereitstellen. Die Beschleunigervorrichtung kann aber auch ein Beschleunigers mit einer Lasereinrichtung zur Strahlerzeugung sein oder ein als "Dielectric Wall" (DWA) bezeichneter Beschleuniger. Das erfindungsgemäße Verfahren und die erfindungsgemäße Bestrahlungsvorrichtung ist jedoch weitgehend unabhängig von der Methode mit der der Therapiestrahl zur Verfügung gestellt wird.

Im Folgenden wird die Erfindung anhand vorteilhafter Ausführungsbeispiele und unter Bezugnahme auf die beigefügte Zeichnung näher erläutert. Gleiche oder ähnliche Gegenstände sind in den Figuren mit gleichen Bezugsziffern bezeichnet. Es zeigt:
- Fig. 1:: eine schematische Darstellung einer Bestrahlungsanlage;
- Fig. 2:: eine schematische Darstellung eines zu bestrahlenden, im Bereich der Lunge eines Patienten sitzenden Tumors, einschließlich in der Nähe liegender kritischer Gewebebereiche;
- Fig. 3:: eine schematische Darstellung von Zielpunkten eines Zielvolumens und von Registrierungspunkten eines zu schützenden Volumens;
- Fig. 4:: eine schematische Darstellung von Zielpunkten eines Zielvolumens und von Registrierungspunkten eines zu schützenden Volumens in einer zweiten Stellung;
- Fig. 5:: eine schematische Darstellung des unterschiedlichen Dosiseintrags eines nadelfeinen lonenstrahls unter Bewegungseinfluss;
- Fig. 6:: ein Beispiel für ein Volumen-Dosis-Diagramm;
- Fig. 7:: eine schematische Darstellung einer Ausführungsform einer Bestrahlungsvorrichtung mit Steuerelementen;
- Fig. 8:: eine schematische Darstellung eines Ablaufs eines Bestrahlungsverfahrens.

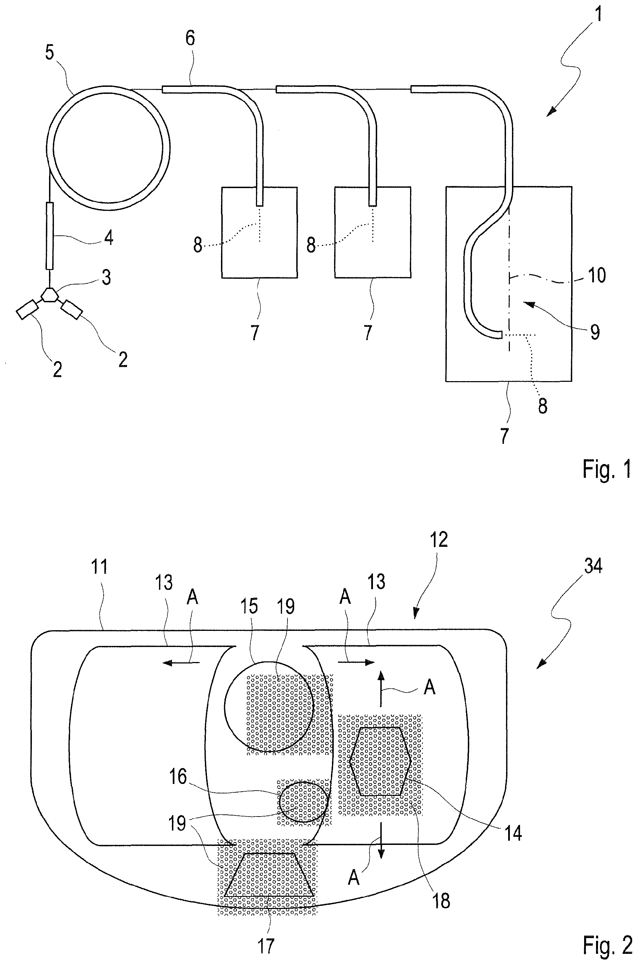

In Fig. 1 ist ein schematischer Überblick über den Aufbau eines Ausführungsbeispiels für eine Bestrahlungsanlage 1 dargestellt, die zum Bestrahlen eines Teilbereichs eines Körpers 34, wie beispielsweise eines tumorerkrankten Gewebes 14 im Körper 34 eines Patienten mit einem Teilchenstrahl 8 dient. Als Teilchen können insbesondere Ionen (üblicherweise Kationen), wie beispielsweise Protonen, Pionen, Heliumionen, Kohlenstoffionen, Neonionen etc., sowie Gemische aus zwei oder mehreren der genannten und gegebenenfalls weiterer Teilchen verwendet werden.

Üblicherweise werden derartige Teilchen in einer Teilchenquelle 2 erzeugt. Im in Fig. 1 dargestellten Ausführungsbeispiel der Bestrahlungsanlage 1 und als Bestrahlungsvorrichtung 1 bezeichnet, sind zwei Teilchenquellen 2, beispielsweise zwei EZR-Ionenquellen vorgesehen, sodass zwei verschiedene Teilchensorten erzeugt werden können. Dadurch ist es möglich, dass innerhalb kurzer Zeit zwischen diesen beiden Teilchensorten umgeschaltet werden kann. Gegebenenfalls ist es auch möglich, ein Gemisch aus beiden Teilchensorten zu erzeugen, indem beide Teilchenquellen 2 gleichzeitig betrieben werden. Um zwischen zwei unterschiedlichen Teilchensorten umschalten zu können, ist ein Schaltmagnet 3 vorgesehen, der zwischen den Teilchenquellen 2 einerseits und einem Vorbeschleuniger 4 andererseits angeordnet ist. Der Schaltmagnet 3 kann darüber hinaus in einem Modus betrieben werden, in dem die von beiden Teilchenquellen 2 erzeugten Teilchen gleichzeitig zum Vorbeschleuniger 4 geführt werden, sodass ein Teilchengemisch erzeugt wird.

Die von einer der, bzw. von beiden Teilchenquellen 2 erzeugten und gegebenenfalls mit dem Schaltmagneten 3 ausgewählten Teilchen werden in dem Vorbeschleuniger 4 auf ein erstes Energieniveau beschleunigt. Der Vorbeschleuniger 4 ist beispielsweise als ein Linearbeschleuniger (LINAC für englisch "LINear ACcelerator") ausgebildet. Nach der Vorbeschleunigung werden die Teilchen in einen Beschleunigerring 5 eingespeist, der beispielsweise als Synchotron oder Zyklotron ausgebildet ist. In dem Beschleuniger 5 werden die Teilchen vom anfänglichen ersten, vergleichsweise niedrigen Energieniveau auf hohe Energien, wie sie zur Bestrahlung erforderlich sind, beschleunigt. Nachdem die Teilchen den Beschleunigerring 5 verlassen haben, führt ein Hochenergiestrahl-Transportsystem 6 den Teilchenstrahl 8 zu einem oder mehreren Bestrahlungsräumen 7.

In einem Bestrahlungsraum 7 werden die beschleunigten Teilchen auf einen zu bestrahlenden Gegenstand 34, insbesondere Körper 34 gerichtet. Dies kann beispielsweise ein Patient sein, der auf einer Liege liegt. Je nach Ausgestaltung erfolgt die Applizierung des Teilchenstrahls 8 auf den Körper 34 aus einer festen Richtung (in sogenannten "fixed beam"-Räumen), oder aber auch von verschiedenen Richtungen aus, wozu eine um eine Achse 10 drehbeweglich rotierbare Gantry 9 vorgesehen ist. Im vorliegend dargestellten Ausführungsbeispiel der Bestrahlungsanlage 1 ist bei insgesamt drei Bestrahlungsräumen 7 nur eine einzelne Gantry 9 vorgesehen, da erfahrungsgemäß bei der Mehrzahl der durchzuführenden Behandlungen eine rotierbare Gantry 9 nicht unbedingt erforderlich ist. Da eine rotierbare Gantry 9 signifikante Kosten verursacht, können hierdurch erhebliche Kosten eingespart werden. Selbstverständlich ist es ebenso möglich, dass keiner der, eine größere Anzahl an oder alle der Bestrahlungsräume 7 mit einer Gantry 9 versehen werden.

Ferner ist in Fig. 1 der Teilchenstrahl 8 angedeutet, der jeweils in den Bestrahlungsräumen 7 freigegeben wird.

In Fig. 2 ist als Beispiel für einen zu bestrahlenden Körper 34 vorliegend ein Querschnitt durch den Brustkorb 11 eines Patienten in Höhe der Lunge 12 dargestellt. In Fig. 2 sind die beiden Lungenflügel 13 zu erkennen, wobei eine der beiden Lungenflügel 13 von einem Tumor 14 befallen ist. Dieser Tumor 14 stellt das von der Bestrahlungsanlage 1 zu bestrahlende Zielvolumen 20 dar. Bei einer Bestrahlung durch die Bestrahlungsanlage 1 gilt es, das Gebiet des Tumors 14 durch Beaufschlagung mit einer ausreichend hohen Dosis, insbesondere Teilchendosis, bevorzugt Ionendosis, zu schädigen bzw. zu zerstören.

Weiterhin sind in Fig. 2 drei kritische Strukturen 15, 16, 17 (Risikostrukturen, fachsprachlich als OAR - "Organ at risk" bezeichnet) dargestellt. Genauer handelt es sich um die Wirbelsäule 17 mit dem darin befindlichen Rückenmark, die Aorta 16 sowie das Mediastinum 15.

Aufgrund der Atmung des Patienten im Laufe der Behandlung kommt es zu einer Bewegung von bestimmten Teilen des Brustkorbs 11. Dies ist in Fig. 2 durch mehrere Pfeile A angedeutet. Insbesondere bewegen sich der Tumor 14, sowie das Mediastinum 15 signifikant. Dies heißt nicht unbedingt, dass sich Wirbelsäule 17 und/oder Aorta 16 nicht bewegen bzw. nicht bewegen können. Üblicherweise ist deren Bewegung jedoch deutlich geringer, als dies bei einem auf einem Lungenflügel 13 befindlichen Tumor 14 bzw. beim Mediastinum 15 der Fall ist.

Insbesondere im Bereich von Weichteilen ist die Bewegung der Gewebestrukturen nicht nur auf eine translatorische Bewegung beschränkt. Vielmehr kommt es hier in aller Regel auch zu Rotationsbewegungen (Drehbewegungen) und/oder zu Dehnungsbewegungen (Dehnung bzw. Stauchung von Gewebestrukturen). Derartige Bewegungen führen zu einer zusätzlichen Verkomplizierung des Problems, insbesondere dann, wenn sich diese Bewegungen zusätzlich zumindest teilweise überlagern.

In Fig. 2 sind darüber hinaus den entsprechenden Körperteilen 14, 15, 16, 17 zugeordnete Raster 18, 19 eingezeichnet. Im Fall des Tumors 14 handelt es sich um ein Zielraster 18, welches im Rahmen einer Rasterscantherapie mit einem Partikelstrahl 18 angefahren wird. Bei den zu schützenden Gewebestrukturen 15, 16, 17 handelt es sich dagegen um ein Registrierungsraster 19, mit dem die (an sich unerwünschte bzw. zu vermeidende) Bestrahlung der entsprechenden Gewebestrukturen 15, 16, 17 registriert wird.

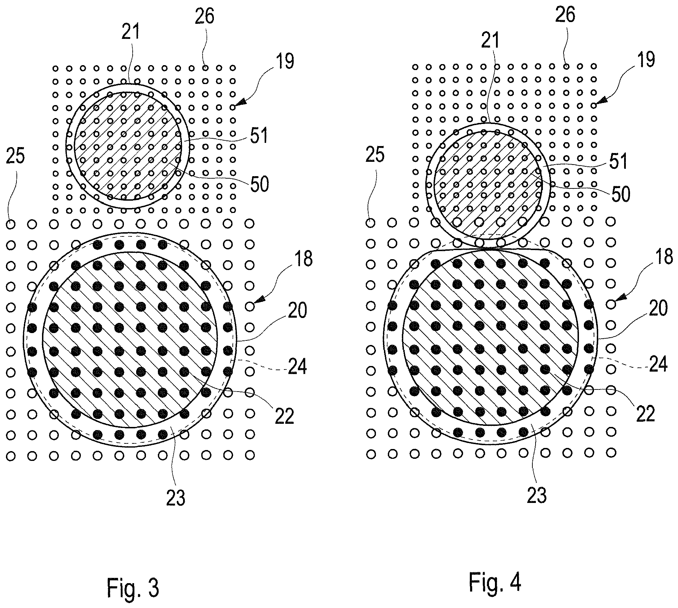

In den Fig. 3 und 4 ist die in Fig. 2 gezeigte Situation nochmals vereinfacht schematisch dargestellt. Aus Vereinfachungsgründen ist dabei in Fig. 3 und in Fig. 4 jeweils nur ein einzelnes zu bestrahlendes Zielvolumen 20, sowie ein einzelnes, (besonders) zu schützendes kritisches Gewebe 21 eingezeichnet. Der innere Bereich 22 des Zielvolumens ist in Fig. 3 und 4 jeweils durch einen von links oben nach rechts unten schraffierten Flächenschnitt dargestellt. Bei dem inneren Bereich 22 handelt es sich beispielsweise um den eigentlichen Tumorbereich 14, also das tatsächlich erkrankte Gewebe. Um den Tumor 14 mit hoher Sicherheit vollständig zu zerstören, ist es dabei üblich, dass um den eigentlichen inneren Bereich 22 herum ein sogenannter Sicherheitssaum 23 vorgesehen wird, der ebenfalls mit einer hohen Strahlendosis belegt wird. Zusätzlich ist in Fig. 3 und 4 eine sogenannte Isodose 24 in Form einer gestrichelte Linie eingezeichnet. Innerhalb der Isodosen 24 liegende Bereiche werden vorliegend mit einer Dosis entsprechend ≥ 95 % der Maximaldosis beaufschlagt.

Weiterhin sind in den Fig. 3 und 4 kritische Gewebebereiche 21 eingezeichnet. In analoger Weise zum Zielvolumen 20 ist auch bei den kritischen Gewebebereichen 21 ein innerer Bereich 25 gegeben, der das eigentliche zu schützende Organgewebe umfasst. Aus Sicherheitsgründen ist der innere Bereich 25 ebenfalls mit einem Sicherheitssaum 26 umgeben. Innerer Bereich 25 und Sicherheitssaum 26 bilden somit das Volumen des kritischen Gewebebereichs 21 aus. Sowohl im Bereich des Zielvolumens 20, als auch im Bereich des kritischen Gewebebereichs 21 ist jeweils ein Raster 18, 19 vorgesehen, welches aus einer Anordnung finiter Volumenelemente 25, 26 ausgebildet ist. Die entsprechenden Raster 18, 19 erstrecken sich in aller Regel dreidimensional. Die einzelnen Volumenelemente, 25, 26 können dabei im Wesentlichen in beliebiger Weise, wie beispielsweise in kubischen, rechteckigen, hexagonalen oder andere Rastern, angeordnet sein.

Im Bereich des Zielvolumens 20 ist ein Zielraster 18 angeordnet, welches aus einer üblicherweise großen Anzahl von Zielpunkten 25 besteht. Jeder der Zielpunkte oder Rasterpunkte 25 kann vom Teilchenstrahl 8 angefahren werden. Hierzu kann der Teilchenstrahl 8 durch Scannermagnete 35, 36 lateral (innerhalb einer sogenannten Isoenergieebene 49) bewegt werden. Um unterschiedliche Isoenergieebenen 49 anfahren zu können, kann die Energie des auf den zu bestrahlenden Körper 34 abgegebenen Teilchenstrahls 8 durch geeignete Vorrichtungen 37 variiert werden. Durch die Anzahl der in einer Zeiteinheit fließenden Teilchen und die Länge des Zeitintervalls, innerhalb dessen der Teilchenstrahl 8 auf den Zielpunkt 25 gerichtet ist, wird für den betreffenden Zielpunkt 25 eine Teilchenzahl, und damit einhergehend eine entsprechende Dosis, definiert. Leere Kreise in Fig. 3 und 4 stellen Zielpunkte 25 dar, auf die der Teilchenstrahl 8 nicht gerichtet wird, oder die nur mit einer geringen Teilchenzahl belegt werden. Ausgefüllte Kreise stellen Zielpunkte 25 dar, die mit einer hohen Teilchenzahl belegt werden.

Im Bereich des kritischen Gewebebereichs 21 ist ein Registrierungsraster 19 vorgesehen. Die finiten Volumenelemente 26, aus denen das Registrierungsraster 19 gebildet ist, werden fachsprachlich üblicherweise als Voxel 26 bezeichnet. Im Unterschied zu den Zielpunkten 25 können im Allgemeinen die Voxel 26 nicht mit dem Teilchenstrahl 8 angefahren werden. Wenn der Teilchenstrahl 8 auf dem Weg zum Zielvolumen 20 jedoch durch den kritischen Gewebebereich 21 hindurch tritt, so wird zwangsläufig eine - wenn auch relativ geringe - Dosis in den entsprechenden Gewebebereichen abgelegt. Diese Dosisbelastung wird auf ein einzelnes Voxel 26 bezogen registriert.

In Fig. 3 und Fig. 4 sind das Zielvolumen 20 und der kritische Gewebebereich 21 unterschiedlich weit voneinander entfernt. Eine derartige Entfernungsänderung kann beispielsweise durch die Atembewegung eines Patienten bewirkt werden. Grenzen Zielvolumen 20 und kritischer Gewebebereich 21 unmittelbar aneinander, so wie dies in Fig. 4 gezeigt ist, so kann eine durch den Teilchenstrahl 8 bewirkte Strahlungsdeposition sehr leicht zu einer unverhältnismäßig hohen und unerwünschten (gegebenenfalls sogar kritischen bzw. bedenklichen) Strahlungsdeposition im Zielvolumen 21 führen. Gemäß dem vorgeschlagenen Verfahren wird jedoch der Strahlungseintrag, der durch den Teilchenstrahl 8 in der erweiterten Umgebung des kritischen Gewebebereichs 21 erfolgt, für jedes einzelne Voxel 26 im Registrierungsraster 19 registriert. Durch Aufsummation ist der gesamte Dosiseintrag pro Voxel 26 ermittelbar. Beim in Fig. 4 dargestellten Beispiel war der Strahlungseintrag im Übergangsbereich zwischen kritischem Gewebebereich 21 und Zielvolumen 20 derart hoch, dass für einzelne Voxel 26 ein Signalwert (Warndosis) überschritten wurde. Dies hatte zur Folge, dass die Bestrahlungsintensität einzelner Zielpunkte 25 im Zielraster 18 verringert wurde (erkennbar daran, dass einzelne Zielpunkte 25 nunmehr als Hohlkreise, und nicht mehr als Vollkreise dargestellt sind). Effektiv hat dies zur Folge, dass im in Fig. 4 dargestellten Ausführungsbeispiel der Sicherheitssaum 23 des zu bestrahlenden Zielvolumens 20 im Nachbarbereich zum kritischen Gewebebereich 21 deutlich reduziert wurde.

Bewegen sich kritischer Gewebebereich 21 und Zielvolumen 20 im Rahmen der Atmung des Patienten jedoch wieder auseinander, so können die betreffenden Zielpunkte 25 wieder in den Scanvorgang mit hoher Dosis einbezogen werden, sodass sich erneut die in Fig. 3 dargestellte Situation ergibt.

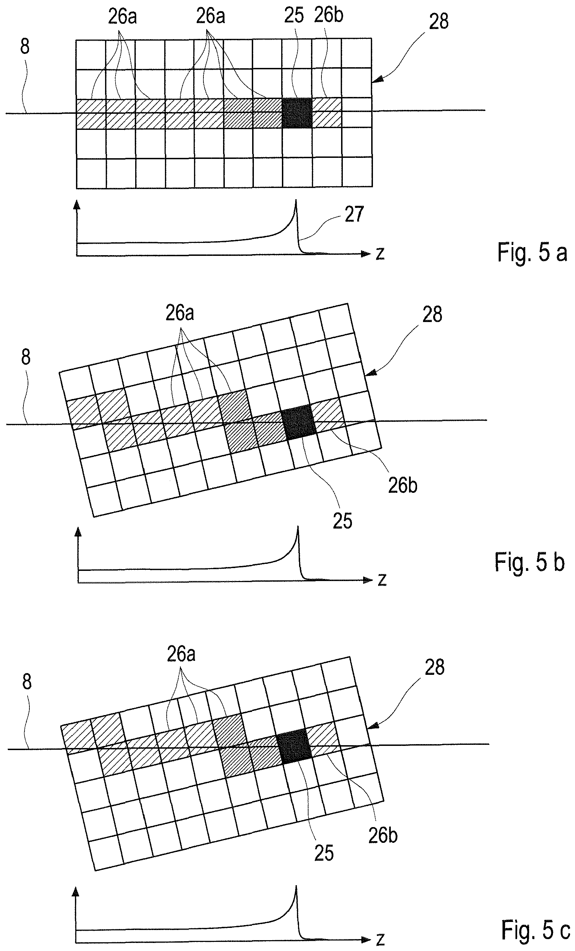

In Fig. 5 wird veranschaulicht, wie die vor dem Zielpunkt 25 befindlichen Voxel durch den einfallenden Teilchenstrahl 8 mit einer gewissen Dosis beaufschlagt werden. Zwar wird bei einem Schwerionenteilchenstrahl 8 der Hauptanteil der Strahlungsenergie im sogenannten Bragg-Peak 27 abgegeben, sodass der Hauptteil der Dosisbeaufschlagung in der Tat im Zielpunkt 25 (bzw. im Zielvolumen 21) freigesetzt wird; dennoch kommt es sowohl in vor dem Zielpunkt 25 gelegenen Voxeln 26a, als auch in nach dem Zielpunkt 25 gelegenen Voxeln 26b (in Fig. 5 ist jeweils nur ein einzelner, nach dem Zielpunkt 25 gelegener Voxel 26 b eingezeichnet) zu einem gewissen Dosiseintrag. Wie man Fig. 5 entnehmen kann, ist der Dosiseintrag in vor dem Zielpunkt 25 gelegenen Voxeln 26a üblicherweise höher als in nach dem Zielpunkt 25 gelegenen Voxeln 26b.

In den Fig. 5b und 5c ist dargestellt, wie eine nicht nur translatorische Bewegung des Gewebeausschnitts 28 dazu führen kann, dass unterschiedliche, an sich nicht vom Teilchenstrahl 8 angefahrenen Voxel 26 einen Dosiseintrag erhalten (siehe insbesondere im Vergleich zwischen Fig. 5b und Fig. 5c). Hierbei stellt Fig. 5b die Situation ohne Anpassung der Strahllage an die Bewegung ("Beam Tracking") dar, während Fig. 5c die Situation mit "Beam Tracking" darstellt. Dies ist daran zu erkennen, dass in Fig. 5c die Bragg-Peak Position wieder im gleichen Voxel wie in Fig. 5a liegt. Insbesondere ist aus Fig. 5 auch ersichtlich, wie eine Rotation des Gewebeausschnitts 28 dazu führen kann, dass völlig unterschiedliche, an sich nicht mit dem Teilchenstrahl 8 beaufschlagte Voxel 26 einen Dosiseintrag erhalten. Insbesondere wird deutlich, dass sich die Dosisbelastung der Voxel 26 ändern kann, selbst wenn "Beam Tracking" benutzt wird. Mit dem Verfahren wird für jedes einzelne der außerhalb des Zielpunkts 25 (bzw. des Zielvolumens 21) gelegenen Voxel 26 online und kontinuierlich der jeweilige Dosiseintrag, und vorzugsweise auch die jeweils kommulierte eingetragene Gesamtdosis registriert. Beim Überschreiten gewisser Werte (wie beispielsweise von Warndosiswerten und/oder Alarmdosiswerten) werden geeignete Maßnahmen ergriffen, wie beispielsweise das "Abschalten" einzelner Zielpunkte 25 (siehe Fig. 4, insbesondere im Vergleich mit Fig. 3).

In Fig. 5b ist der Teilchenstrahl 8 aufgrund einer Bewegung (beispielsweise Atembewegung) des Zielvolumens 20/des Zielpunkts 25 kurzfristig vom "eigentlich" geplanten Zielpunkt 25 weggewandert. In Fig. 5b ist daher eine (kurzfristige) Strahlabweichung von einer Voxellänge gegeben. Dementsprechend werden auch unterschiedliche Voxel 26 und Zielpunkte 25 bestrahlt. In Fig. 5c hat das "tracking"-Verfahren wieder gegriffen, nunmehr wird erneut der "eigentlich" geplante Zielpunkt 25 bestrahlt.

Sowohl bei der in Fig. 5b, als auch bei der in Fig. 5c gezeigten Situation (selbstverständlich auch bei der in Fig. 5a gezeigten Situation) wird der Dosisbeitrag für alle tatsächlich (mehr oder weniger stark) bestrahlten Voxel 26 beziehungsweise Zielpunkte 25 erfasst. Die "Fehlbestrahlung" gemäß Fig. 5b kann dabei durch eine geeignet korrigierte Strahlführung/Bestrahlungsplanung im nächsten beziehungsweise in den nächsten Scandurchgängen des Teilchenstrahls 8 berücksichtigt und vorzugsweise ausgeglichen werden. Insbesondere kann die notwendige Kompensation der "Fehlbestrahlung" online und im selben und/oder einem oder mehreren späteren Bestrahlungsdurchgang bzw. Bestrahlungsdurchgängen erfolgen.

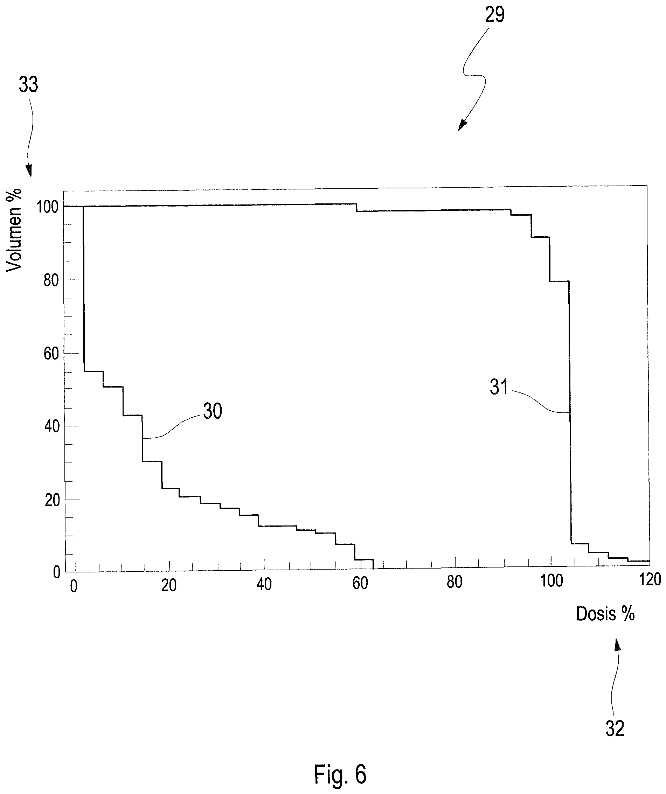

In Fig. 6 ist dargestellt, dass die Signalwerte (insbesondere Warnwerte 30 und/oder Maximalwerte 31) nicht notwendigerweise als fixer Zahlenwert vorliegen müssen. Stattdessen ist es auch möglich (und im Übrigen auch bevorzugt), dass sich die Höhe der eine Signalausgabe bewirkenden Dosiswerte 30, 31 mit der Größe des bestrahlten Volumens (wie beispielsweise dem bestrahlten Volumen eines kritischen Organs) ändert. Derartige Verhältnisse zueinander sind in Form von sogenannten Volumen-Dosis-Diagrammen (DVH) 29 an sich bekannt. Ein typisches Beispiel ist in Fig. 6 gezeigt. Dabei ist die Dosis in % längs der Abszisse 32 aufgetragen, während der bestrahlte relative und/oder zu bestrahlende Volumenanteil längs der Ordinate 33 aufgetragen ist. Die Kurve 31 bezeichnet hierbei beispielsweise das DVH für das zu bestrahlende Zielvolumen. Die Kurve 30 bezeichnet die Dosis, die das zu schützende Volumen maximal erhalten darf. Somit stellt die Kurve 30 einen Maximalwert für das zu schützende Volumen dar. Aus dem Maximalwert kann dann der Warnwert bestimmt werden. Wie man dem DVH entnehmen kann, sind höhere Dosiswerte zulässig, wenn sich diese auf ein relativ kleines Volumengebiet beschränken. Wird das betreffende Gebiet jedoch flächig, insbesondere im Wesentlichen vollständig bestrahlt, so sind die zulässigen Werte sowohl zur Auslösung eines Warnsignals (Warnwert), als auch zur Auslösung eines Maximalsignals (Maximalwert) zum Teil deutlich niedriger. Beispielsweise ist es auch üblich Maximalwerte für Anteile der Risikoorgane zu definieren. Ein "V20" Maximalwert von 40 Gy setzt zum Beispiel fest, dass die 20% des betreffenden Risikoorgans mit der höchsten Dosisbelastung 40 Gy zumindest nicht vollständig überschreiten dürfen. Der Warnwert kann insbesondere zwischen 50 % und 90 % des Maximalwertes liegen.

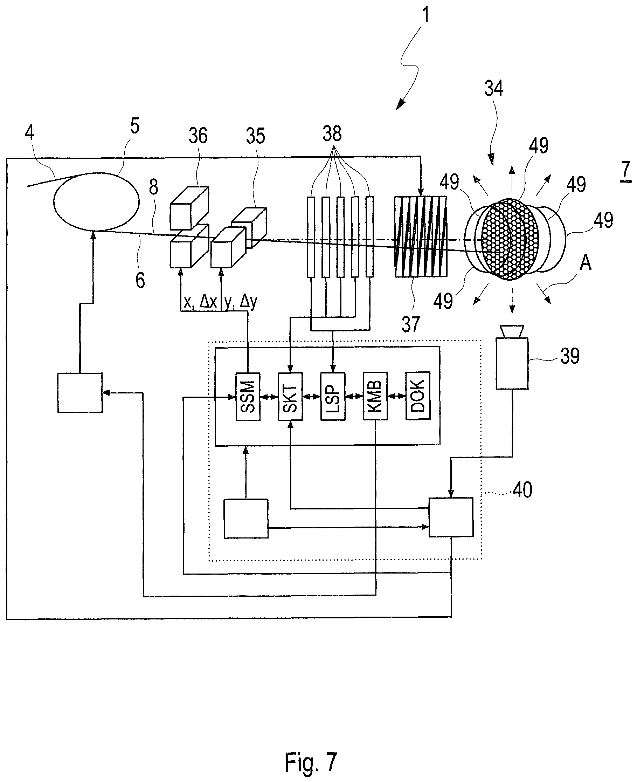

In Fig. 7 ist die in Fig. 1 bereits dargestellte Bestrahlungsanlage 1 nochmals in schematischer Darstellung dargestellt. Obgleich es sich in Fig. 1 und Fig. 7 um die gleiche Bestrahlungsanlage 1 handelt, ist bei den jeweiligen Schemazeichnungen der Fokus auf unterschiedliche Komponenten der Bestrahlungsanlage 1 gelegt. Der von der Bestrahlungsanlage 1 erzeugte, aus dem Beschleunigerring 5 extrahierte Teilchenstrahl 8 wird über das Hochenergiestrahl-Transportsystem 6 dem zu bestrahlenden Körper 34, der sich im Bestrahlungsraum 7 befindet, zugeführt. Der zu bestrahlende Körper 34 umfasst dabei die in Fig. 2 näher dargestellten zu bestrahlenden Körperregionen 14 bzw. die nicht zu bestrahlenden Körperregionen (einschließlich der besonders empfindlichen Gewebestrukturen 15, 16, 17). Wie durch Pfeile A angedeutet ist, bewegt sich der Körper 34, insbesondere auch in sich (Verschiebung, Verdrehung, Dehnung und Stauchung der einzelnen Geweberegionen 28 in sich und untereinander).

Damit sämtliche Zielpunkte 25 des Zielrasters 18, welches im Bereich des zu bestrahlenden Zielvolumens 20 liegt (vergleiche Fig. 3, 4) angefahren werden können, sind zunächst ein vertikaler Scannermagnet 36 sowie ein horizontaler Scannermagnet 35 vorgesehen. Mit diesen kann eine laterale Verschiebung des Wirkorts des Teilchenstrahls 8 (also in X- und Y-Richtung, wenn Z die Richtung des Teilchenstrahls 8 darstellt) innerhalb einer Isoenergieebene 49 des zu bestrahlenden Körpers 34 erreicht werden. Eine Variation des Wirkpunkts des Partikelstrahls 8 in Z-Richtung erfolgt durch eine Teilchenstrahlenergieanpassvorrichtung 37, welche vorliegend als Energieabsorber 37 ausgebildet ist. Der in Fig. 7 dargestellte Energieabsorber 37 besteht aus einer Mehrzahl von jeweils keilförmig ausgebildeten Körpern aus einem energieabsorbierendem Material. Die in Fig. 7 oben und unten befindlichen Keile sind jeweils zu einem Keilblock miteinander verbunden. Über Stellmotoren (vorliegend nicht dargestellt) können die beiden Keilblöcke der Energieabsorbereinrichtung 37 aufeinander zu bzw. voneinander weg verschoben werden. Je nach Stellung der beiden Keilblöcke relativ zueinander hat der Teilchenstrahl 8 einen unterschiedlich langen Weg durch das energieabsorbierende Material, aus dem die Keile der Keilblöcke gebildet sind, zurück zu legen. Dementsprechend viel Energie verliert er beim Durchgang durch die Energieabsorbereinrichtung 37, sodass mit der Energieabsorbereinrichtung 37 (innerhalb bestimmter Grenzen) die Energie des auf den Körper 34 treffenden Teilchenstrahls 8 schnell geändert werden kann.

Zwischen den Scannermagneten 35 und 36 sowie der Energieabsorbereinrichtung 37 sind noch unterschiedliche Detektoren 38 vorgesehen, die vorliegend zum Teil als gasgefüllte Vieldrahtkammern, zum Teil dagegen als sogenannte Ionisationskammern ausgebildet sind. Mit Hilfe der Detektoren 38 kann sowohl die Lage des Teilchenstrahls 8, als auch dessen Energie (vor dem Durchgang durch die Energieabsorbereinrichtung 37) ermittelt werden. In Verbindung mit dem Stellsignal der Energieabsorbereinrichtung 37 (wobei die Position der Keilblöcke beispielsweise durch geeignete Messgeräte gemessen werden kann) kann dann auch die Energie des auf den Körper 34 einfallenden Teilchenstrahls 8 bestimmt werden.

Selbstverständlich ist es auch möglich, Scannermagneten 35, 36, die Absorbereinrichtung 37 und die Detektoren 38 in einer unterschiedlichen Reihenfolge anzuordnen. Bereits mit den beschriebenen Steuersignalen und Messwerten ist die Steuereinheit 40 in der Lage, die auf die unterschiedlichen Voxel 26 im Körper 34 eingebrachte Dosis (insbesondere die applizierte Dosis) zu ermitteln. Dies betrifft nicht nur die Dosis, die in zu bestrahlende Zielvolumina 21 eingebracht wird, sondern insbesondere auch die Dosis die (an sich unerwünschterweise) in kritische Gewebebereiche 21 und/oder in sonstige Gewebebereiche eingebracht wird. Die entsprechende Information liegt dabei ortsaufgelöst vor, da die eingetragene Dosis pro Voxel 26 registriert wird. Durch Aufsummation der einzelnen Dosisbeiträge kann die im Laufe des Bestrahlungsvorgangs akkumulierte Dosis pro Voxel 26 in sämtlichen unterschiedlichen Gewebebereichen zu jedem Zeitpunkt im Wesentlichen verzögerungsfrei ausgelesen werden. Hierzu kann insbesondere auch das Verfahren verwendet werden, welches in der Patentanmeldung mit der Überschrift "Verfahren und Vorrichtung zur Steuerung der Dosisapplikation bei der Bestrahlung" am 26. November 2009 von der gleichen Anmelderin und unter dem anmelderseitigen Aktenzeichen P 286 beim Deutschen Patent- und Markenamt als deutsche Patentanmeldung mit dem amtlichen Aktenzeichen DE 10 2009 055 902.7 hinterlegt wurde.

Überschreitet die jeweilige akkumulierte eingebrachte Dosis einen bestimmten Grenzwert (Warnwert/Maximalwert), so wird ein entsprechendes Signal von der Steuereinrichtung 40 ausgegeben. Der Signalwert kann dabei auch von der Größe des bestrahlten Volumen abhängig sein (siehe Fig. 6).

Um die Genauigkeit der Bestrahlung weiter zu erhöhen, ist bei der vorliegend dargestellten Bestrahlungsanlage 1 zusätzlich ein PET 39 (PET für Photonen-Emissions-Tomograph) vorgesehen. Mit dem PET ist es insbesondere möglich, die im Körper 34 deponierte Dosis zu messen. Falls der PET 39 ortsaufgelöst und/oder zeitaufgelöst messen kann, können die entsprechenden Messwerte auch ortsaufgelöst (direkt) und/oder zeitaufgelöst verwendet werden. Selbst wenn der PET 39 nicht ortsaufgelöst messen kann, ist auf indirektem Wege eine ortsaufgelöste Messung (mit eingeschränkter Genauigkeit) unter Verwendung der sonstigen Mess- und Steuersignale möglich.

Anstatt oder zusätzlich zu einer direkten Dosismessung wird die Bewegung des Zielvolumens und des zu schützenden Volumens des Gegenstandes gemessen. Hieraus kann, vorzugsweise in Verbindung mit einer 4-dimensionalen Bestrahlungsplanung (4D-Bestrahlungsplanung), online während der Bestrahlung die eingebrachte Dosis bestimmt werden.

Wie bereits erwähnt, reagiert die Steuereinrichtung 40 unterschiedlich, je nachdem ob - und gegebenenfalls welches - Signal generiert/ausgegeben wird. Wird ein Maximalsignal generiert/ausgegeben, so kann die Steuereinrichtung 40 beispielsweise eine Schnellabschaltung des Teilchenstrahls 8 bewirken. Wird dagegen nur ein Warndosiswert erreicht bzw. überschritten, so kann die Steuereinrichtung 40 die zeitlich nachfolgenden Teile des Rasterscanverfahrens geeignet anpassen. Dies kann beispielsweise durch Gating, durch Rescanning und/oder durch Modifikation der Bestrahlungsplanung erfolgen. Da eine vollständige Neuberechnung einer Bestrahlungsplanung in der Regel eine signifikante Zeitdauer erfordert (typischerweise im Bereich von mehreren Stunden), ist es möglich, die Bestrahlungsplanung lediglich zu modifizieren. Hierbei wird insbesondere das vorstehend genannte Verfahren der DE 10 2009 055 902.7 eingesetzt.

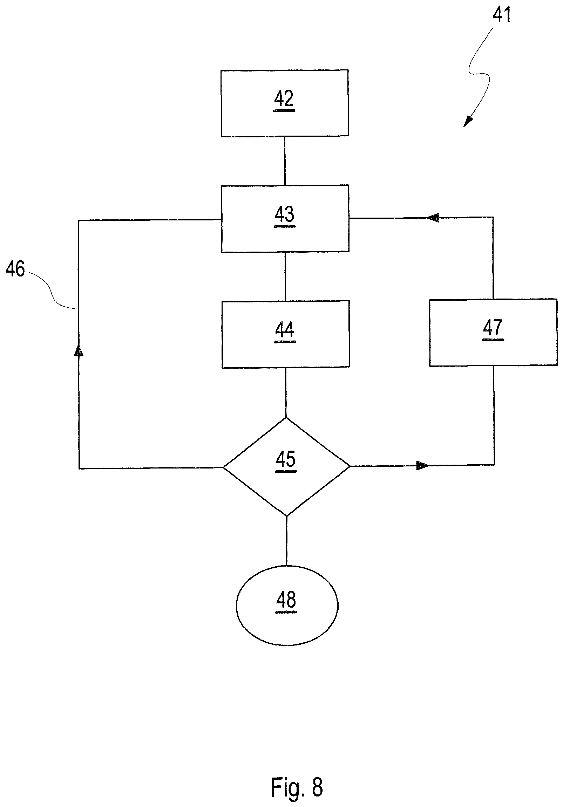

In Fig. 8 ist schließlich noch ein möglicher Ablaufplan des vorgeschlagenen Bestrahlungsverfahrens 41 in schematischer Form dargestellt. Beim Bestrahlungsverfahren 41 wird zunächst von einer Soll-Bestrahlung 42 ausgegangen. Diese Soll-Bestrahlung 42 kann beispielsweise in Form einer an sich bekannten Bestrahlungsplanung vorliegen. Basierend auf den Werten der Soll-Bestrahlung 42 werden die einzelnen Komponenten der Bestrahlungsanlage 1 geeignet angesteuert 43, sodass in einem bzw. in einer Mehrzahl von Zielpunkten 25 bzw. Zielvolumina 20 eine entsprechende Dosis eingebracht wird. Unter Verwendung der Soll-Ansteuerung der Bestrahlungsanlage 1, und/oder von Messwerten der Bestrahlungsanlage 1 (insbesondere des Teilchenstrahls 8 und/oder von "direkten Messungen" 39) wird der Dosiseintrag aufgrund des erzeugten Teilchenstrahls 8 ortsaufgelöst bestimmt 44. Dabei erfolgt die Bestimmung 44 vorzugsweise für sämtliche Bereiche des bestrahlten Körpers 34, insbesondere (aber nicht unbedingt nur) für die kritischen, zu schützenden Volumen 21 im zu bestrahlenden Körper 34. Anschließend werden die soeben ermittelten Dosiswerte (vorzugsweise ebenfalls ortsaufgelöst) überprüft 45. Je nach Ergebnis der Überprüfung 45 wird das Bestrahlungsverfahren 41 unterschiedlich weitergeführt. Wurde kein Signaldosiswert (insbesondere kein Warndosiswert und/oder kein Maximaldosiswert) überschritten, so wird das Verfahren ohne Änderung weiter geführt 46. Das heißt, dass die Bestrahlung entsprechend der ursprünglichen Sollbestrahlung 42 (bzw. einer gegebenenfalls früher modifizierten Sollbestrahlung 47) fortgeführt. Dies kann auch dazu führen, dass das Bestrahlungsverfahren 41 beendet wird, weil beispielsweise gemäß der ursprünglichen Soll-Bestrahlung 42 die im Tumor 14 zu deponierende Dosis bereits erreicht wurde.

Ist dagegen ein Warndosiswert überschritten, so wird die geplante Bestrahlung (also die ursprüngliche Soll-Bestrahlung 42 oder eine bereits früher modifizierte Planung 47) modifiziert. Die Modifikation erfolgt hierbei bevorzugt online während der Bestrahlung, wobei der zu bestrahlende Gegenstand mit dem modifizierten Behandlungsplan bestrahlt wird. Die Modifikation hat dabei insbesondere zum Ziel, dass nach Möglichkeit kein bzw. ein nur geringer weiterer Anstieg in den Bereichen des zu bestrahlenden Körpers 34 erfolgt, die zur Auslösung des Warnsignals führten und/oder bereits eine relativ hohe eingebrachte Dosis (welche insbesondere über einem Warndosiswert liegt) akkumuliert haben.

Wird dagegen im Überprüfungsschritt 45 festgestellt, dass bereits eine Maximaldosis erreicht wurde, so wird das Verfahren 41 sofort abgebrochen 48. Eine spätere Behandlung kann dann vorzugsweise erst nach einer Freigabe durch einen behandelnden Arzt erfolgen, wobei der weitere Therapienutzen bzw. die weitere Therapiegefährdung im Einzelnen abzuwägen ist.

Die in Zusammenhang mit den Figuren 1 bis 8 beschriebenen Verfahrensschritte und/oder Vorrichtungsdetails können auch in anderen als den ausdrücklich beschriebenen Kombinationen und/oder alleinstehend eingesetztund realisiert werden.

### Bezugszeichenliste

1. Bestrahlungsanlage
2. Partikelquelle
3. Schaltmagnet
4. Vorbeschleuniger
5. Beschleunigerring
6. Hochenergiestrahl-Transportsystem
7. Bestrahlungsraum
8. Partikelstrahl
9. Gantry
10. Achse der Gantry
11. Brustkorb
12. Lunge
13. Lungenflügel
14. Tumor
15. Mediastinum
16. Aorta
17. Wirbelsäule
18. Zielraster
19. Registrierungsraster
20. Zielvolumen
21. Kritischer Gewebebereich
22. Innerer Bereich von 20
23. Sicherheitssaum von 20
24. Isodose
25. Zielpunkt
26. Voxel
27. Bragg-Peak
28. Gewebeausschnitt
29. Volumen-Dosis-Diagramm
30. Warnwert
31. Maximalwert
32. Abszisse
33. Ordinate
34. Körper
35. Horizontaler Scanner
36. Vertikaler Scanner
37. Energieabsorbtionseinrichtung
38. Detektor
39. PET
40. Steuereinrichtung
41. Bestrahlungsverfahren
42. Soll-Bestrahlung
43.Ansteuerung Bestrahlungsanlage
44. Bestimmung Dosiseintrag
45. Überprüfung
46. Keine Änderung
47.Änderung Soll-Bestrahlung
48.Abbruch
49. Isoenergieebene
50. Innerer Bereich von 21
51. Sicherheitssaum von 21

## Patentansprüche

1. Bestrahlungsvorrichtung (1) zur Bestrahlung von Gegenständen (34) mit einem Scanningverfahren mit einem Hadronenstrahl und umfassend eine Steuervorrichtung (40) sowie eine Bewegungsmessvorrichtung (39), wobei der Gegenstand (34) zumindest ein zu bestrahlendes Zielvolumen (14, 20) sowie zumindest ein zu schützendes Volumen (10, 16, 17, 21) aufweist, wobei für das zu bestrahlende Zielvolumen (14, 20) ein Zielraster (18) und für das zu schützende Volumen (10, 16, 17, 21) ein Registrierungsraster (19) vorgesehen sind, wobei für das zu schützende Volumen (10, 16, 17, 21) zumindest ein Signaldosiswert (30, 31) definiert ist, wobei die Bewegung des zu bestrahlenden Zielvolumens (14, 20) und des zu schützenden Volumens (10, 16, 17, 21) gemessen werden, wobei während der Bestrahlung (43) des Gegenstands (34) in jedem Volumenelement (26) des Registrierungsrasters (19) die in das zu schützende Volumen (10, 16, 17, 21) eingebrachte Dosis ermittelt wird (44) und von der Steuervorrichtung (40) zumindest ein Signal ausgegeben (47, 48) wird, sobald in zumindest einem Punkt (26) des Registrierungsrasters (19) die in das zu schützende Volumen (10, 16, 17, 21) eingebrachte Dosis zumindest einen Signaldosiswert (30, 31) überschreitet.

2. Bestrahlungsvorrichtung (1) nach Anspruch 1, **dadurch gekennzeichnet, dass** es sich bei zumindest einem Signaldosiswert (30, 31) um einen Warndosiswert (30) und/oder eine nicht zu überschreitende Maximaldosis (31) handelt.

3. Bestrahlungsvorrichtung (1) nach Anspruch 1 oder 2, **dadurch gekennzeichnet, dass** das ausgegebene Signal (47, 48) einen Abbruch des Bestrahlungsvorgangs (48) bewirkt und/oder eine Unterbrechung des Bestrahlungsvorgangs (47) und/oder eine Änderung (47) von zumindest Teilen des noch durchzuführenden Bestrahlungsvorgangs, wie insbesondere eine Verringerung der in zumindest Teilen des zu schützenden Volumens (10, 16, 17, 21) und/oder in zumindest Teilen des zu bestrahlenden Zielvolumen (14, 20) einzubringenden Dosis, bewirkt.

4. Bestrahlungsvorrichtung (1) nach einem der vorangehenden Ansprüche, **dadurch gekennzeichnet, dass** es sich bei der eingebrachten Dosis zumindest zeitweise und/oder zumindest teilweise um die applizierte Dosis und/oder um die deponierte Dosis handelt.

5. Bestrahlungsvorrichtung (1) nach einem der vorangehenden Ansprüche, **dadurch gekennzeichnet, dass** die Ermittlung der in das zu schützende Volumen (10, 16, 17, 21) eingebrachten Dosis und/oder die Ermittlung der in das zu bestrahlende Zielvolumen (14, 20) eingebrachten Dosis zumindest teilweise und/oder zumindest zeitweise unter Verwendung zumindest eines im betreffenden Volumen (10, 14, 16, 17, 20, 21) und/oder zumindest eines am Strahl (8) gemessenen Messwerts (38, 39) erfolgt, wobei es sich bei dem am Strahl (8) gemessenen Messwert (38, 39) insbesondere um die Strahlposition, die Strahlgröße, die Strahlform, die Strahlintensität und/oder die Strahlenergie handelt.

6. Bestrahlungsvorrichtung (1) nach einem der vorangehenden Ansprüche, **dadurch gekennzeichnet, dass** die Position des zu bestrahlenden Zielvolumens (14, 20) und des zu schützenden Volumens (10, 16, 17, 21) unter zumindest teilweiser Verwendung bildgebender Verfahren (39) gemessen wird.

7. Bestrahlungsvorrichtung (1) nach einem der vorangehenden Ansprüche, **dadurch gekennzeichnet, dass** die Bestrahlungsvorrichtung dazu hergerichtet ist, daß die Bestrahlung in Form eines Rasterscanvorgangs erfolgt.

8. Bestrahlungsvorrichtung (1) nach einem der vorangehenden Ansprüche, **gekennzeichnet durch** zumindest eine Strahlmessvorrichtung (37, 38), bevorzugt eine Strahlpositionsmessvorrichtung (38), Strahlintensitätsmessvorrichtung (38) und/oder Strahlenergiemessvorrichtung (37), welche bevorzugt zumindest bereichsweise als Ionisationskammervorrichtung (38) ausgebildet ist.

## Claims

1. An irradiation device (1) for irradiating objects (34) using a scanning method with a hadron beam and comprising a control device (40) and a movement measuring device (39), wherein the object (34) has at least one target volume to be irradiated (14, 20) and at least one volume to be protected (10,16,17,21), wherein a target raster (18) is provided for the target volume to be irradiated (14, 20) and a register raster (19) is provided for the volume to be protected (10, 16, 17, 21), wherein at least one signal dose value (30, 31) is defined for the volume to be protected (10,16,17,21), wherein the movements of the target volume to be irradiated (14, 20) and of the volume to be protected (10, 16, 17, 21) are measured, wherein during irradiation (43) of the object (34) the dose introduced into the volume to be protected (10, 16, 17, 21) is determined (44) in each volume element (26) of the register raster (19), and at least one signal is output (47, 48) by the control device (40) as soon as the dose introduced into the volume to be protected (10,16,17, 21) exceeds at least one signal dose value (30, 31) in at least one point (26) of the register raster (19).

2. The irradiation device (1) according to claim 1, **characterized in that** at least one signal dose value (30, 31) is a warning dose value (30) and/or a maximum dose (31) that must not be exceeded.

3. The irradiation device (1) according to claim 1 or 2, **characterized in that** the output signal (47, 48) causes a termination of the irradiation process (48) and/or an interruption of the irradiation process (47) and/or a modification (47) of at least parts of the irradiation process still to be performed, such as in particular a reduction in the dose to be introduced into at least parts of the volume to be protected (10, 16, 17, 21) and/or into at least parts of the target volume to be irradiated (14, 20).

4. The irradiation device (1) according to any one of the preceding claims, **characterized in that** the introduced dose is at least temporarily and/or at least partially the applied dose and/or the deposited dose.

5. The irradiation device (1) according to any one of the preceding claims, **characterized in that** the determination of the dose introduced into the volume to be protected (10, 16, 17, 21) and/or the determination of the dose introduced into the target volume to be irradiated (14, 20) is performed at least partially and/or at least temporarily using at least one measured value (38, 39) measured in the volume in question (10, 14, 16, 17, 20, 21) and/or measured at the beam (8), wherein the measured value (38, 39) measured at the beam (8) in particular is one of the beam position, the beam size, the beam shape, the beam intensity, and/or the beam energy.

6. The irradiation device (1) according to any one of the preceding claims, **characterized in that** the position of the target volume to be irradiated (14, 20) and of the volume to be protected (10, 16, 17, 21) is measured at least partially using imaging methods (39).

7. The irradiation device (1) according to any one of the preceding claims, **characterized in that** the irradiation device is adapted to perform the irradiation in the form of a raster scanning process.

8. The irradiation device (1) according to any one of the preceding claims, **characterized by** at least one beam measuring device (37, 38), preferably a beam position measuring device (38), beam intensity measuring device (38), and/or beam energy measuring device (37), which is preferably at least partially provided in the form of an ionization chamber device (38).

## Revendications

1. Dispositif d'irradiation (1) destiné à l'irradiation d'objets (34) par un procédé de balayage avec un faisceau de hadrons et comprenant un dispositif de commande (40) ainsi qu'un dispositif de mesure de mouvement (39), l'objet (34) présentant au moins un volume cible à irradier (14, 20) ainsi qu'au moins un volume à préserver (10, 16, 17, 21), une trame cible (18) étant prévue pour le volume cible à irradier (14, 20) et une trame d'enregistrement (19) étant prévue pour le volume à préserver (10, 16, 17, 21), au moins une valeur de dose de signal (30, 31) étant définie pour le volume à préserver (10, 16, 17, 21), le mouvement du volume cible à irradier (14, 20) et du volume à préserver (10, 16, 17, 21) étant mesuré, sachant que lors de l'irradiation (43) de l'objet (34), on détermine, dans chaque élément de volume (26) de la trame d'enregistrement (19), la dose introduite dans le volume à préserver (10, 16, 17, 21), et que le dispositif de commande (40) émet au moins un signal (47, 48) dès que la dose introduite dans le volume à préserver (10, 16, 17, 21) dépasse au moins une valeur de dose de signal (30, 31) dans au moins un point (26) de la trame d'enregistrement (19).

2. Dispositif d'irradiation (1) selon la revendication 1, **caractérisé en ce qu'**au moins une valeur de dose de signal (30, 31) est une valeur de dose d'avertissement (30) et/ou une dose maximale (31) à ne pas dépasser.

3. Dispositif d'irradiation (1) selon la revendication 1 ou 2, **caractérisé en ce que** le signal émis (47, 48) provoque l'arrêt du processus d'irradiation (48) et/ou provoque une interruption du processus d'irradiation (47) et/ou une modification (47) au moins de parties du processus d'irradiation restant à effectuer, par exemple notamment une réduction de la dose à introduire au moins dans des parties du volume à préserver (10, 16, 17, 21) et/ou au moins dans des parties du volume cible à irradier (14, 20).

4. Dispositif d'irradiation (1) selon une des revendications précédentes, **caractérisé en ce que** la dose introduite est au moins par instants et/ou au moins en partie la dose appliquée et/ou la dose déposée.

5. Dispositif d'irradiation (1) selon une des revendications précédentes, **caractérisé en ce que** la détermination de la dose introduite dans le volume à préserver (10, 16, 17, 21) et/ou la détermination de la dose introduite dans le volume cible à irradier (14, 20) est effectuée au moins en partie et/ou au moins par instants en utilisant au moins une valeur de mesure (38, 39) mesurée dans le volume (10, 14, 16, 17, 20, 21) concerné et/ou au moins une valeur de mesure mesurée sur le faisceau (8), la valeur de mesure (38, 39) mesurée sur le faisceau (8) étant notamment la position du faisceau, la taille du faisceau, la forme du faisceau, l'intensité du faisceau et/ou l'énergie du faisceau.

6. Dispositif d'irradiation (1) selon une des revendications précédentes, **caractérisé en ce que** la position du volume cible à irradier (14, 20) et du volume à préserver (10, 16, 17, 21) est mesurée en utilisant au moins en partie des procédés d'imagerie (39).

7. Dispositif d'irradiation (1) selon une des revendications précédentes, **caractérisé en ce que** le dispositif d'irradiation est agencé de manière à ce que l'irradiation s'effectue sous la forme d'un processus de balayage de trame.

8. Dispositif d'irradiation (1) selon une des revendications précédentes, **caractérisé en ce qu'**il comporte au moins un dispositif de mesure de faisceau (37, 38), de préférence un dispositif de mesure de position de faisceau (38), un dispositif de mesure d'intensité de faisceau (38) et/ou un dispositif de mesure d'énergie de faisceau (37), qui est réalisé de préférence au moins en partie comme dispositif à chambre d'ionisation (38).
